# EUROPEAN PATENT SPECIFICATION

(11) **EP 2 555 757 B1**
(45) Date of publication and mention of the grant of the patent: **25.05.2016**
(21) Application number: 11714907.0
(22) Date of filing: 07.04.2011
(51) Int. Cl.: A61K 9/20, A61K 31/4402

(54) **ATAZANAVIR SULFATE FORMULATIONS WITH IMPROVED PH EFFECT**
ATAZANAVIR-SULFAT-FORMULIERUNGEN MIT VERBESSERTEM PH-EFFEKT
FORMULATIONS DE SULFATE D'ATAZANAVIR AVEC UN EFFET PH AMÉLIORÉ

(30) Priority: 09.04.2010 US 322487 P
(43) Date of publication of application: 13.02.2013
(73) Proprietor: Bristol-Myers Squibb Holdings Ireland, 6312 Steinhausen (CH)
(72) Inventor: NIKFAR, Faranak, New Brunswick New Jersey 08903 (US); HUSSAIN, Munir Alwan, New Brunswick New Jersey 08903 (US); QIAN, Feng, New Brunswick New Jersey 08903 (US)
(74) Representative: Reitstötter Kinzebach
(86) International application number: PCT/US2011/031526
(87) International publication number: WO 2011/127244

(56) References cited:
- WO-A1-95/09614
- WO-A1-2005/034948
- WO-A2-2009/084036
- Karen Dahri ET AL: "Atazanavir and Acid-Lowering Therapy", Canadian Journal of Hospital Pharmacy, 1 January 2008 (2008-01-01), pages 21-29, XP55021068, Retrieved from the Internet: URL:http://www.cjhp-online.ca/index.php/cj hp/article/viewFile/7/6# [retrieved on 2012-03-06]

## Description

### FIELD OF THE INVENTION

The present invention relates to pharmaceutical compositions and medical uses thereof.

### BACKGROUND OF THE INVENTION

Human immunodeficiency virus (HIV) has been identified as the etiological agent responsible for acquired immune deficiency syndrome (AIDS), a serious disease characterized by destruction of the immune system and the inability to fight off life threatening opportunistic infections.

U.S. Patent No. 5,849,911 issued to Fässler et al. discloses a series of azapeptide HIV protease inhibitors (which includes atazanavir) which have the structure 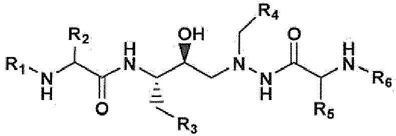 wherein
R₁ is lower alkoxycarbonyl,
R₂ is secondary or tertiary lower alkyl or lower alkylthio-lower alkyl,
R₃ is phenyl that is unsubstituted or substituted by one or more lower alkoxy radicals, or C₄-C₈ cycloalkyl,
R₄ is phenyl or cyclohexyl each substituted in the 4-position by unsaturated heterocyclyl that is bonded by way of a ring carbon atom, has from 5 to 8 ring atoms, contains from 1 to 4 hetero atoms selected from nitrogen, oxygen, sulfur, sulfinyl (-SO-) and sulfonyl (-SO₂-) and is unsubstituted or substituted by lower alkyl or by phenyl-lower alkyl,
R₅, independently of R₂, has one of the meanings mentioned for R₂, and
R₆, independently of R₁, is lower alkoxycarbonyl, or a salt thereof, provided that at least one salt-forming group is present which includes various pharmaceutically acceptable acid addition salts thereof.

U.S. Patent No. 6,087,383 issued to Singh et al. discloses the bisulfate salt of the azapeptide HIV protease inhibitor known as atazanavir which has the structure 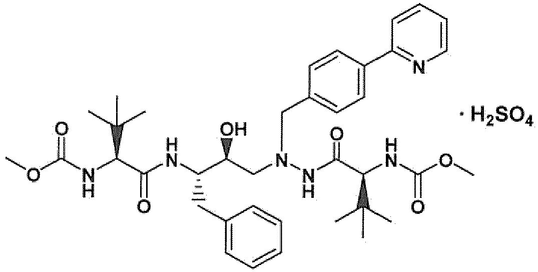 (referred to herein as "atazanavir bisulfate" or "atazanavir sulfate").

U.S. Patent Publication No. US20050256202A1, published November 17, 2005, discloses processes for preparing the HIV protease inhibitor atazanavir bisulfate and novel forms thereof.

WO2009/002821 published December 31, 2008 discloses compressed tablets containing atazanavir sulfate, optionally with another active agents, e.g., anti-HIV agents, granules that contain atazanavir sulfate and an intragranular lubricant that can be used to make the tablets, compositions comprising a plurality of the granules, processes for making the granules and tablets, and methods of treating HIV.

WO2009/084036 describes a solid dosage form comprising ritonavir and atazanavir, or pharmaceutically acceptable salts of these agents, and a pharmaceutical carrier, a surfactant and optionally other pharmaceutically acceptable excipients.

Atazanavir is commercially available as a prescription medicine from Bristol-Myers Squibb Company, New York, under the tradename REYATAZ^{®} (atazanavir sulfate) for the treatment of HIV. Approved in 2003 by the U.S. Food and Drug Administration, REYATAZ^{®} (atazanavir sulfate) is currently available in the form of 100 milligram ("mg"), 150 mg, 200 mg, and 300 mg capsules. Patient demand for REYATAZ^{®} (atazanavir sulfate) has been substantial and continues to grow.

H₂ receptor antagonists are a class of drugs used to block the action of histamine on parietal cells in the stomach, decreasing the production of acid by these cells. Typical H₂ antagonists include cimetidine, ranitidine, famotidine, and nizatidine. Proton pump inhibitors are a group of drugs whose main action is a pronounced and long-lasting reduction of gastric acid production. Typical proton pump inhibitors include benzimidazole derivatives and imidazopyridine derivatives. An antacid includes any substance, generally a base or basic salt, which neutralizes stomach acidity. It has been observed that atazanavir sulfate in the capsule dosage form exhibits pH-dependent absorption and oral absorption of the drug can be reduced when the drug is administered with H-2 antagonists, proton pump inhibitors or antacids. Accordingly, improved dosage forms, product formulations and manufacturing processes are desired that can decrease the pH dependence of the dosage form and enhance the oral absorption of atazanavir sulfate when administered with H-2 antagonists, proton pump inhibitors, or antacids.

### SUMMARY OF THE INVENTION

New formulations and manufacturing processes for atazanvir sulfate tablets and capsules are described below to mitigate the pH sensitivity of the dosage forms. When prepared in a tableted form, the tablets can optionally include other active ingredients, e.g., other antiretroviral agents.

The present invention is directed to a composition in the form of a compressed tablet comprising atazazavir sulfate, GS 9350 (cobicistat), an acidifying agent and a precipitation retardant polymer. The inclusion of the acidifying agent can help to maintain low microenvironmental pH during dissolution, and may be enhanced by the inclusion of a precipitation retardant polymer. The precipitation retardant polymer comprised by the tablet of the invention is selected from
polyvinylpyrrolidone (hereinafter referred to "PVP"), polyvinylpyrrolidone-vinyl acetate copolymer (hereinafter referred to "PVP-VA"), hydroxypropylcellulose, hydroxypropyl methylcellulose, hydroxypropylmethylcellulose acetate succinate (or hypromellose acetate succinate) (hereinafter referred to as "HPMC-AS") and mixtures thereof.

Further, methods of preparing the compositions of the invention by wet and dry granulation process, or either hot-melt extrusion or spray drying processes are provided.

### DETAILED DESCRIPTION OF THE INVENTION

In accordance with the present invention, the method in which the atazanavir sulfate is prepared is not critical. For example, the atazanavir sulfate is present as Form A, Form E3 or Pattern C, preferably in particular in pharmaceutically M/53486-EP acceptable form. Often, the crystalline forms of atazanavir and salts thereof are in substantially pure form. These forms are described, for example, in U.S. Patent Publication No. US20050256202A1, published November 17, 2005. The term "pharmaceutically acceptable", as used herein, refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio. The term "substantially pure", as used herein, means a compound having a chemical purity of at least about 90 wt%, preferably at least about 95 wt%, more preferably at least about 98 wt% of the compound and less than about 10 wt%, preferably less than about 5 wt%, and more preferably less than about 2 wt% of other compounds having a different chemical structure than the compound.
In one suitable method, atazanavir in the form of its free base may be prepared by treating a solution of a protected triamine salt of the structure 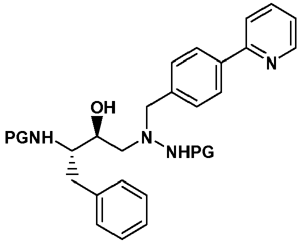 (where PG represents a protecting group such as t-butyloxycarbonyl (Boc) or trifluoroacetyl, preferably Boc, with an acid, preferably hydrochloric acid (where Boc is used), or a base (where trifluoroacetyl is used) in the presence of an organic solvent such as methylene chloride, tetrahydrofuran, or methanol, which solvent is preferably methylene chloride, at a temperature within the range from about 25 to about 50 °C, preferably from about 30 to about 40 °C, to form the triamine acid salt, preferably the hydrogen chloride salt of the structure 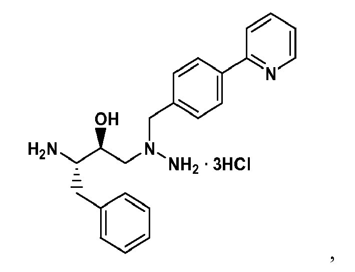 and without isolating the triamine acid salt, reacting the triamine acid salt with an active ester of an acid of the structure 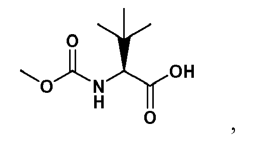 preferably the active ester of the structure 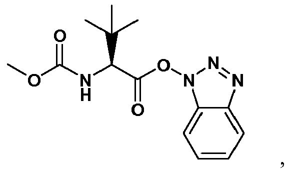 in the presence of a base such as K₂HPO₄ diisopropylethylamine, N-methylmorpholine, sodium carbonate, or potassium carbonate, preferably K₂HPO₄, in the presence of an organic solvent such as methylene chloride, a mixture of ethyl acetate and butyl acetate, acetonitrile or ethyl acetate, preferably methylene chloride, at a temperature within the range from about 25 to about 50 °C, preferably from about 30 to about 40 °C to form atazanavir free base.

The protected triamine starting material may be prepared by reacting the epoxide 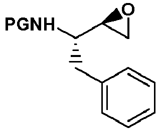 where PG is preferably Boc such as N-(tert-butyloxycarbonyl)-2(S)-amino-1-phenyl-3(R)-3,4-epoxy-butane, with the hydrazine carbamate 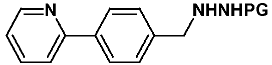 where PG is preferably Boc in the presence of isopropyl alcohol or other alcohol such as ethanol or butanol.

One suitable method for preparing Form A crystals of atazanavir sulfate salt, a modified cubic crystallization technique is employed wherein atazanavir free base is dissolved in an organic solvent in which the atazanavir sulfate salt is substantially insoluble and includes acetone, a mixture of acetone and N-methyl pyrrolidone, ethanol, a mixture of ethanol and acetone and the like, to provide a solution having a concentration of atazanavir free base within the range from about 6.5 to about 9.7% by weight, preferably from about 6.9 to about 8.1% by weight atazanavir free base.

The solution of atazanavir free base is heated at a temperature within the range from about 35 to about 55 °C, preferably from about 40 to about 50 °C, and reacted with an amount of concentrated sulfuric acid (containing from about 95 to about 100% H₂SO₄) to react with less than about 15%, preferably from about 5 to less than about 12%, more preferably from about 8 to about 10% by weight of the total atazanavir free base. Thus, the starting solution of atazanavir free base will be initially reacted with less than about 15%, preferably from about 5 to about 12%, by weight of the total amount of sulfuric acid to be employed. During the reaction, the reaction mixture is maintained at a temperature within the range from about 35 to about 55 °C, preferably from about 40 to about 50 °C.

The reaction is allowed to continue for a period from about 12 to about 60 minutes, preferably from about 15 to about 30 minutes.

The reaction mixture is seeded with crystals of Form A atazanavir sulfate employing an amount of seeds within the range from about 0.1 to about 80% by weight, preferably from about 3 to about 8% by weight, based on the weight of atazanavir free base remaining in the reaction mixture while maintaining the reaction mixture at a temperature within the range from about 35 to about 55 °C, preferably from about 40 to about 50 °C.

The reaction is allowed to continue until crystallization begins. Thereafter, sulfuric acid is added in multiple stages at an increasing rate according to the cubic equation as described in U.S. Patent Publication No. US20050256202A1, published November 17, 2005 to form atazanavir sulfate which upon drying produces Form A crystals.

The crystal particle size and morphology of the atazanavir sulfate salt formed are dependent on the addition rate of the sulfuric acid, which determines the crystallization rate. It has been found that a modified "cubic" crystallization technique (acid added at an increasing rate according to a cubic equation) provides relatively larger, more well defined atazanavir sulfate crystals, along with a narrower particle size range and fewer fines, than a constant addition rate crystallization. The slow initial acid flow rate has been shown to favor crystal growth over secondary nucleation. Thus, as the surface area increases with particle size, the seed bed is able to accept the increasing acid flow rate without inducing secondary nucleation. The slow initial addition rate allows time for the crystals to grow larger, increasing the mean size. The cubic crystallization provides a less compressible filter cake, which aids in effective cake deliquoring and washing, as well as giving a more easily dried product with fewer hard lumps than the constant addition rate crystallized product.

Pattern C material may be prepared, for example, by exposing Form A crystals to water followed by drying. Pattern C material may also be formed by exposing crystals of Form A to high relative humidity of greater than about 95% RH, preferably from about 95 to about 100% RH (water vapor), for at least 24 hours, preferably from about 24 to about 48 hours. Pattern C material may also be prepared by wet granulating atazanavir sulfate Form A to produce granules of atazanavir sulfate and then drying the granules.

The Form E3 may be prepared, for example, by slurrying atazanavir free base in ethanol, treating the slurry with concentrated sulfuric acid employing a molar ratio of acid: free base with the range from about 1:1 to about 1.1:1, heating the resulting solution at from about 30 to about 40 °C, seeding the solution with ethanol wet E3 crystals of atazanavir sulfate, treating the mixture with heptane (or other solvent such as hexane or toluene), filtering, and drying to yield atazanavir sulfate Form E3 (triethanol solvate). The seeding step will employ an amount of seeds to effect formation of E3 crystals, for example a molar ratio of atazanavir sulfate E-3 seeds:free base within the range from about 0.02:1 to about 0.04:1.

Further details concerning the preparation of the atazanavir sulfate suitable for use in accordance with the present invention are described, for example, in U.S. Patent Publication No. US20050256202A1, published November 17, 2005.

The present invention contemplates the use of any pharmaceutically acceptable ingredients, such as, for example, lubricants, disintegrants, binders, fillers (also referred to as "compression aids"), surfactants, film coatings, and solvents. Examples of some of these ingredients are set forth below and are described in more detail in the Handbook of Pharmaceutical Excipients, Second Edition, Ed. A. Wade and P. J. Weller, 1994, The Pharmaceutical Press, London, England. The selection and amounts of such ingredients to be used in accordance with the present invention are not critical and can be determined by one skilled in the art.

Examples of lubricants suitable for use in accordance with the invention, include but are not limited to, magnesium stearate, zinc stearate, calcium stearate, stearic acid, palmitic acid, sodium stearyl fumarate, sodium benzoate, sodium lauryl sulfate, glyceryl monostearate, glyceryl palmitostearate, hydrogenated castor oil, hydrogenated vegetable oil, mineral oil, carnauba wax, and polyethylene glycol. In accordance with the invention, ingredients also referred to as "glidants" are intended to be included within the scope of lubricants. Examples include, but are not limited to, silicon dioxide, calcium silicate, calcium phosphate and talc.

Examples of disintegrants suitable for use in accordance with the invention, include but are not limited to, croscarmellose sodium, crospovidone, potato starch, pregelatinized starch, corn starch, sodium starch glycolate, microcrystalline cellulose, powdered cellulose, methylcellulose, carboxymethylcellulose calcium, carboxymethylcellulose sodium, alginic acid, colloidal silicon dioxide, guar gum, magnesium aluminum silicate, polyacrilin potassium and sodium alginate.

Examples of binders suitable for use in accordance with the invention, include but are not limited to, acacia, carbomer, dextrin, gelatin, guar gum, hydrogenated vegetable oil, methylcellulose, ethyl cellulose, cellulose acetate, hydroxyethyl cellulose, hydroxypropyl cellulose, hydroxypropyl methylcellulose, carboxymethylcellulose sodium, glucose, lactose, magnesium aluminum silicate, maltodextrin, polymethacrylates, povidone, polyvinyl pyrrolidone, corn starch, pregelatinized starch, alginic acid, sodium alginate, zein, carnauba wax, paraffin, spermaceti, polyethylenes and microcrystalline wax.

Examples of fillers suitable for use in accordance with the invention, include but are not limited to, microcrystalline cellulose, lactose, sucrose, starch, pregelatinized starch, dextrose, dextrates, dextrin, mannitol, fructose, xylitol, sorbitol, corn starch, modified corn starch, inorganic salts such as calcium carbonate, magnesium carbonate, magnesium oxide, calcium phosphate, dicalcium phosphate, tribasic calcium phosphate, calcium sulfate, dextrin/dextrates, maltodextrin, compressible sugars, confectioner's sugar, glyceryl palmitostearate, hydrogenated vegetable oil, kaolin, maltodextrin, polymethacrylates, potassium chloride, sodium chloride, sucrose, sugar spheres and talc.

Examples of acidifying agents suitable for use in accordance with the invention, include but are not limited to citric acid, fumaric acid, tartaric acid, and ascorbic acid.

Precipitation retardant agents suitable for use in accordance with the invention are polyvinylpyrrolidone, polyvinylpyrrolidone-vinyl acetate, hydroxypropyl cellulose, hydoxypropyl methylcellulose, hydroxypropylmethylcellulose acetate succinate and mixtures thereof.

In accordance with the invention, when the ingredients are incorporated prior to granulation, they are referred to as "intragranular", i.e., within the granule. When the ingredients are incorporated after granulation, they are referred to as "extragranular".

In one aspect of the invention, atazanvir sulfate in crystalline Form A is mixed with one or more acidifying agents such as, for example, citric acid, tartaric acid, fumaric acid, ascorbic acid and optionally one or more precipitation retardant polymers such as polyvinylpyrrolidone, hydroxypropyl cellulose, hydroxypropyl methylcellulose, hydroxypropyl methylcellulose acetate succinate, and optionally with one or more suitable fillers and optionally with one or more disintegrants. The blend is wet or dry granulated yielding the intragranular portion. The granules are typically mixed with the extragranular ingredients, e.g., fillers, disintegrants, and lubricants, and optionally acidifying agents and precipitation retardant agents, and compressed into tablets.

Typically the granule comprises from about 10-80 percent by weight ("w/w %") of the atazanavir sulfate, more typically from about 40 to 70 w/w % of the atazanavir sulphate, based on the total weight of the granule.

The granules also comprise from about 5 to 30 w/w % acidifying agent, more typically from about 10 to 25 w/w % based on the total weight of the granule.

The granule may also comprise, for example, from about 1 to 20 w/w %, based on the total weight of the granule, of a disintegrant.

The granule may optionally further comprise, for example, from about 0 -15 w/w % precipitation retardant agent, based on the total weight of the granule.

The granule may further comprise, for example, from about 0 to 20 w/w %, based on the total weight of the granule, of a filler.

Typically, the compressed tablets contain about 20-70 w/w % granules, more typically from about 30 to 60 w/w % of the granules, based on the total weight of the tablet. The extragranular composition may optionally include 5-20 w/w % acidifying agents, and 1-5 w/w % precipitation retardant agent. Other extragranular excipients such as fillers, disintegrants, and lubricants may also be incorporated therein.

In one aspect, a composition of atazanavir sulfate can be prepared by a process comprising: (a) blending atazanavir sulfate and an acidifying agent to form a first blend; and (b) granulating the first blend to form a granulated blend. Typically, the process further comprises: (c) blending the granulated blend with an extragranular ingredient to form a second blend; and (d) compressing the second blend to form a tablet.

In order to prepare the composition herein above described, a wet or dry granulation process available to the skilled artisan can be employed. When the wet granulation process is employed, the atazanavir sulfate may exist in the form of "Pattern C" in the compressed tablets.

In another aspect, a composition of atazanavir sulfate can be prepared by a process comprising: (a) combining atazanavir sulfate and a polymer to form a first combination; (b) extruding the first combination to form an extrudate; (c) blending the extrudate with an acidifying agent to form a second combination; and (d) compressing the second combination to form a tablet.

For example, in this aspect, an amorphous solid dispersion composition of atazanavir sulfate and one or more polymers such as PVP and PVP-VA may be prepared by a process of hot melt extrusion by heating a composition of the atazanavir sulfate and the polymer(s) to a temperature of about 160-190°C. The extrudate can be formulated with additional acidifying agent and optionally precipitation retardant polymers. Other tableting ingredients such as fillers, disintegrants, and lubricants, can be added and the final blend is compressed into tablets. In this aspect of the invention, the extrudate may, for example, contain about 10-60 w/w % of the amorphous atazanvir sulfate together with about 90-40 w/w % of PVP-VA and/or PVP. More typically, the extrudate contains about 20-45 w/w % of atazanvir sulfate, with about 80-55 w/w % of PVP and/or PVP-VA. Typically, the compressed tablets contain about 20-60 w/w % extrudate, more typically from about 30-55 w/w % of the extrudate, based on the total weight of the compressed tablet. The extrudate is typically blended with about 10-30 w/w % acidifying agents and optionally 1-5 w/w % of precipitation retarding excipients. Other excipients such as, for example, fillers, disintegrants, and lubricants, may also be incorporated therein the composition.

In another aspect, a composition of atazanavir sulfate can be prepared by a process comprising: (a) providing a solution of atazanavir sulfate and an acidifying agent dissolved in a solvent; (b) spray drying the solution to form particles; (c) blending the particles with an extragranular ingredient to form a spray-dried blend; and (d) compressing the spray dried blend to form a tablet.

For example, in this aspect, an amorphous solid dispersion composition of atazanavir sulfate, a copolymer based on ethylene oxide and propylene oxide such as, for example, Pluronic^{®} copolymers available from BASF Corporation, Florham Park, New Jersey, an acidifying agent, and optionally a precipitation retardant polymer is provided, and can be prepared, for example, by a spray drying process using a solvent such as, for example, methanol or methanol/water solution (e.g., about 5-10 % w/w). The inlet temperature of the spray dry apparatus is typically about 60-100 °C, while the outlet temperature is about 30-60 °C. The spray-dried material typically has a particle size corresponding to the cumulative volume percentages of 90%, i.e., d90, of under about 50 micrometers ("µm"). Often, no further milling is required; however, the spray-dried material may be milled, if desired. In this aspect, the spray-dried material contains about 10-60 w/w % of the amorphous atazanvir sulfate together with about 10-40 %w/w of acidifying agent and optionally about 5-30 w/w % of a precipitation retardant polymer. More typically, the particles contain about 20-45 w/w % of atazanvir sulfate, with about 15-30 %w/w of acidifying agent and optionally about 10-20 w/w % of a precipitation retardant polymer. The spray-dried material can be

blended with 10-30 w/w % acidifying agents and optionally 1-5 w/w % of precipitation retarding excipients. Other excipients such as fillers, disintegrants, and lubricants, may also be incorporated therein the composition.

In another aspect of the invention, one or more other agents having anti-HIV activity, and/or the ability to enhance the pharmacokinetics of the atazanavir, is included in the compressed tablet in addition to cobicistat. As used herein, the term "anti-HIV activity" means the agent has efficacy against the HIV virus. Other agents may be selected, for example, from the group consisting of nucleoside HIV reverse transcriptase inhibitors, non-nucleoside HIV reverse transcriptase inhibitors, HIV protease inhibitors, HIV fusion inhibitors, HIV attachment inhibitors, CCR5 inhibitors, CXCR4 inhibitors, HIV budding or maturation inhibitors, and HIV integrase inhibitors.

Another aspect of the invention is the compressed tablet wherein the other agent is a nucleoside HIV reverse transcriptase inhibitor selected from the group consisting of abacavir, didanosine, emtricitabine, lamivudine, stavudine, tenofovir, zalcitabine, and zidovudine, or a pharmaceutically acceptable salt thereof. A preferred combination with atazanavir is wherein the other agents are tenofovir disoproxil fumarate and emtricitabine. A typical dosage for the drug Truvada™ (emtricitabine - tenofovir disoproxil fumarate) is emtricitabine 200 mg plus tenofovir 300 mg one tablet once per day. A typical dosage for the drug Epzicom™ (abacavir-lamivudine) is abacavir sulfate 600 mg and lamivudine 300 mg. Suitable dosages for combination therapy with atazanavir can be determined by those skilled in the art.

Another aspect of the invention is the compressed tablet wherein the other agent is a non-nucleoside HIV reverse transcriptase inhibitor selected from the group consisting of delavirdine, efavirenz, nevirapine and UK 453061 or a pharmaceutically acceptable salt thereof.

Another aspect of the invention is the compressed tablet wherein the other agent is a HIV protease inhibitor selected from the group consisting of amprenavir, indinavir, lopinavir, nelfinavir, ritonavir, saquinavir and fosamprenavir, or a pharmaceutically acceptable salt thereof. Ritonavir is a preferred drug to be used in combination with atazanavir sulfate as another agent having anti-HIV activity. However, ritonavir is more commonly used as a boosting agent for another drug, e.g., atazanavir. When given as a protease inhibitor booster, the dosing typically ranges from 100-400 mg twice daily or, if used as a part of a once-daily regimen, 100-200 mg once-daily.

Another aspect of the invention is the compressed tablet wherein the other agent is a HIV fusion inhibitor selected from enfuvirtide or T-1249, or a pharmaceutically acceptable salt thereof.

Another aspect of the invention is the compressed tablet wherein the other agent is a CCR5 inhibitor selected from the group consisting of maraviroc, Sch-C, Sch-D, TAK-220, PRO-140, PF-232798 and UK-427,857, or a pharmaceutically acceptable salt thereof.

Another aspect of the invention is the compressed tablet wherein the other agent is the CXCR4 inhibitor AMD-3100 or a pharmaceutically acceptable salt thereof.

Another aspect of the invention is the compressed tablet wherein the other agent is the budding or maturation inhibitor PA-457, or a pharmaceutically acceptable salt thereof.

Another aspect of the invention is the compressed tablet wherein the other agent is the integrase inhibitor raltegravir, or a pharmaceutically acceptable salt thereof. The chemical name of the potassium salt is N-[(4-fluorophenyl)methyl]-1,6-dihydro-5-hydroxy-1-methyl-2-[1-methyl-1-[[(5-methyl-1,3,4-oxadiazol-2-yl)carbonyl]amino]ethyl]-6-oxo-4-pyrimidinecarboxamide monopotassium salt. Raltegravir is described, for example, in WO 2003/035077 published May 1, 2003 and Drugs of the Future 2007, 32(2): 118-122, Y Wang., et al. Typical dosages for raltegravir in monotherapy are 100, 200, 400, and 600 mg given twice daily. Suitable dosages for combination therapy with atazanavir can be determined by those skilled in the art.

Another aspect of the invention is the compressed tablet wherein the other agent is elvitegravir.

Another aspect of the invention is the compressed tablet wherein the other agents are emtricitabine and tenofovir disoproxil fumarate.

Table 1 includes some agents useful in treating AIDS and HIV infection which may by suitable for use in accordance with this invention as the other agents having anti-HIV activity, as well as other drugs that may be co-administered.

**Table 1. ANTIVIRALS**

| Drug Name | Manufacturer | Indication |
|---|---|---|
| 097 (non-nucleoside reverse transcriptase inhibitor) | Hoechst/Bayer | HIV infection, AIDS, ARC |
| Amprenavir 141 W94 GW 141 (protease inhibitor) | Glaxo Wellcome | HIV infection, AIDS, ARC |
| Abacavir (1592U89) GW 1592 (RT inhibitor) | Glaxo Wellcome | HIV infection, AIDS, ARC |
| Acemannan | Carrington Labs (Irving, TX) | ARC |
| Acyclovir | Burroughs Wellcome | HIV infection, AIDS, ARC, in combination with AZT |
| AD-439 | Tanox Biosystems | HIV infection, AIDS, ARC |
| AD-519 | Tanox Biosystems | HIV infection, AIDS, ARC |
| Adefovir dipivoxil AL-721 | Gilead Sciences Ethigen (Los Angeles, CA) | HIV infection, ARC, PGL HIV positive, AIDS |
| Alpha Interferon HIV in combination w/Retrovir | Glaxo Wellcome | Kaposi's sarcoma |
| Ansamycin LM 427 | Adria Laboratories (Dublin, OH) Erbamont (Stamford, CT) | ARC |
| Antibody which Neutralizes pH Labile alpha aberrant Interferon | Advanced Biotherapy Concepts (Rockville, MD) | AIDS, ARC |
| AR177 | Aronex Pharm | HIV infection, AIDS, ARC |
| Beta-fluoro-ddA | Nat'1 Cancer Institute | AIDS-associated diseases |
| BMS-232623 (CGP-73547) (protease inhibitor) | Bristol-Myers Squibb/ Novartis | HIV infection, AIDS, ARC |
| BMS-234475 (CGP-61755) (protease inhibitor) | Bristol-Myers Squibb/ Novartis | HIV infection, AIDS, ARC |
| CI-1012 | Warner-Lambert | HIV-1 infection |
| Cidofovir | Gilead Science | CMV retinitis, herpes, papillomavirus |
| Curdlan sulfate | AJI Pharma USA | HIV infection |
| Cytomegalovirus Immune globin | Medlmmune | CMV retinitis |
| Cytovene | Syntex | Sight threatening |
| Ganciclovir | | CMV peripheral, CMV retinitis |
| Delaviridine (RT inhibitor) | Pharmacia-Upj ohn | HIV infection, AIDS, ARC |
| Dextran Sulfate | Ueno Fine Chem. Ind. Ltd. (Osaka, Japan) | AIDS, ARC, HIV positive asymptomatic |
| ddC Dideoxycytidine | Hoffman-La Roche | HIV infection, AIDS, ARC |
| ddI Dideoxyinosine | Bristol-Myers Squibb | HIV infection, AIDS, ARC; combinationwith AZT/d4T |
| DMP-450 (protease inhibitor) | AVID (Camden, NJ) | HIV infection, AIDS, ARC |
| Efavirenz (DMP 266) (-)6-Chloro-4-(S)-cyclopropylethynyl-4(S)-trifluoro-methyl-1,4-dihydro-2H-3,1-benzoxazin-2-one, STOCRINE (non-nucleoside RT inhibitor) | DuPont Merck | HIV infection, AIDS, ARC |
| EL10 | Elan Corp, PLC (Gainesville, GA) | HIV infection |
| Emtricitabine (Emtriva^{®}) (reverse transcriptase inhibitor) | Gilead | HIV infection, AIDS |
| Famciclovir | Smith Kline | herpes zoster, herpes simplex |
| FTC (reverse transcriptase inhibitor) | Emory University | HIV infection, AIDS, ARC |
| GS 840 (reverse transcriptase inhibitor) | Gilead | HIV infection, AIDS, ARC |
| HBY097 (non-nucleoside reverse transcriptaseinhibitor) | Hoechst Marion Roussel | HIV infection, AIDS, ARC |
| Hypericin | VIMRx Pharm. | HIV infection, AIDS, ARC |
| Recombinant Human Interferon Beta | Triton Biosciences (Almeda, CA) | AIDS, Kaposi's sarcoma, ARC |
| Interferon alfa-n3 | Interferon Sciences | ARC, AIDS |
| Indinavir | Merck | HIV infection, AIDS, ARC, asymptomatic HIV positive, also in combination with AZT/ddI/ddC |
| ISIS 2922 | ISIS Pharmaceuticals | CMV retinitis |
| KNI-272 | Nat'l Cancer Institute | HIV-associated diseases |
| Lamivudine, 3TC (reverse transcriptase inhibitor) | Glaxo Wellcome | HIV infection, AIDS, ARC, also with AZT |
| Lobucavir | Bristol-Myers Squibb | CMV infection |
| Nelfinavir (protease inhibitor) | Agouron Pharmaceuticals | HIV infection, AIDS, ARC |
| Nevirapine (RT inhibitor) | Boeheringer Ingleheim | HIV infection, AIDS, ARC |
| Novapren | Novaferon Labs, Inc. (Akron, OH) | HIV inhibitor |
| Peptide T Octapeptide Sequence | Peninsula Labs (Belmont, CA) | AIDS |
| Trisodium Phosphonoformate | Astra Pharm. Products, Inc. | CMV retinitis, HIV infection, other CMV infections |
| PNU-140690 (protease inhibitor) | Pharmacia Upjohn | HIV infection, AIDS, ARC |
| Probucol | Vyrex | HIV infection, AIDS |
| RBC-CD4 | Sheffield Med. Tech (Houston, TX) | HIV infection, AIDS, ARC |
| Ritonavir (protease inhibitor) | Abbott | HIV infection, AIDS, ARC |
| Saquinavir (protease inhibitor) | Hoffmann-LaRoche | HIV infection, AIDS, ARC |
| Stavudine; d4T Didehydrodeoxy-thymidine | Bristol-Myers Squibb | HIV infection, AIDS, ARC |
| Valaciclovir | Glaxo Wellcome | Genital HSV & CMVinfections |
| Virazole Ribavirin | Viratek/ICN (Costa Mesa, CA) | asymptomatic HIV-positive, LAS, ARC |
| VX-478 | Vertex | HIV infection, AIDS, ARC |
| Zalcitabine | Hoffmann-LaRoche | HIV infection, AIDS, ARC, with AZT |
| Zidovudine; AZT | Glaxo Wellcome | HIV infection, AIDS, ARC, Kaposi's sarcoma, in combination with other therapies |
| Tenofovir disoproxil, fumarate salt (Viread^{®}) (reverse transcriptase inhibitor) | Gilead | HIV infection, AIDS |
| Combivir^{®} (reverse transcriptase inhibitor) | GSK | HIV infection, AIDS |
| abacavir succinate (or Ziagen^{®}) (reverse transcriptase inhibitor) | GSK | HIV infection, AIDS |
| Fuzeon (Enfuvirtide, T-20) | Roche/Trimeris | HIV infection, AIDS, viral fusion inhibitor |
| Trizivir^{®} | | HIV infection, AIDS |
| Kaletra^{®} | Abbott | HIV infection, AIDS, ARC |

**IMMUNOMODULATORS**

| Drug Name | Manufacturer | Indication |
|---|---|---|
| AS-101 | *Wyeth-Ayerst* | AIDS |
| Bropirimine | Pharmacia Upjohn | Advanced AIDS |
| Acemannan | Carrington Labs, Inc. (Irving, TX) | AIDS, ARC |
| CL246,738 | American Cyanamid Lederle Labs | AIDS, Kaposi's sarcoma |
| EL10 | Elan Corp, PLC (Gainesville, GA) | HIV infection |
| FP-21399 | Fuki ImmunoPharm | Blocks HIV fusion with CD4+ cells |
| Gamma Interferon | Genentech | ARC, in combination w/TNF (tumor necrosis factor) |
| Granulocyte Macrophage Colony Stimulating Factor | Genetics Institute Sandoz | AIDS |
| Granulocyte Macrophage Colony Stimulating Factor | Hoechst-Roussel Immunex | AIDS |
| Granulocyte Macrophage Colony Stimulating Factor | Schering-Plough | AIDS, combination w/AZT |
| HIV Core Particle Immunostimulant | Rorer | Seropositive HIV |
| IL-2 Interleukin-2 | Cetus | AIDS, in combination w/AZT |
| IL-2 Interleukin-2 | Hoffman-LaRoche Immunex | AIDS, ARC, HIV, in combination w/AZT |
| IL-2 Interleukin-2 (aldeslukin) | Chiron | AIDS, increase in CD4 cell counts |
| Immune Globulin Intravenous (human) | Cutter Biological (Berkeley, CA) | Pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute | AIDS, ARC |
| Alpha-2 Interferon | Schering Plough | Kaposi's sarcoma w/AZT, AIDS |
| Methionine-Enkephalin | TNI Pharmaceutical (Chicago, IL) | AIDS, ARC |
| MTP-PE Muramyl-Tripeptide Granulocyte Colony Stimulating Factor | Ciba-Geigy Corp. Amgen | Kaposi's sarcoma AIDS, in combination w/AZT |
| Remune | Immune Response Corp. | Immunotherapeutic |
| rCD4 Recombinant Soluble Human CD4 | Genentech | AIDS, ARC |
| rCD4-IgG hybrids | | AIDS, ARC |
| Recombinant Soluble Human CD4 | Biogen | AIDS, ARC |
| Interferon Alfa 2a | Hoffman-La Roche in combination w/AZT | Kaposi's sarcoma, AIDS, ARC |
| SK&F 106528 Soluble T4 | Smith Kline | HIV infection |
| Thymopentin | Immunobiology Research Institute (Annandale, NJ) | HIV infection |
| Tumor Necrosis Factor; TNF | Genentech | ARC, in combination w/gamma Interferon |

**ANTI-INFECTIVES**

| Drug Name | Manufacturer | Indication |
|---|---|---|
| Clindamycin with Primaquine | Pharmacia Upjohn | PCP |
| Fluconazole | Pfizer | Cryptococcal meningitis, candidiasis |
| Pastille Nystatin Pastille | Squibb Corp. | Prevention of oral candidiasis |
| Ornidyl Eflornithine | Merrell Dow | PCP |
| Pentamidine Isethionate (IM & IV) | LyphoMed (Rosemont, IL) | PCP treatment |
| Trimethoprim | | Antibacterial |
| Trimethoprim/sulfa | | Antibacterial |
| Piritrexim | Burroughs Wellcome | PCP treatment |
| Pentamidine Isethionate for Inhalation | Fisons Corporation | PCP prophylaxis |
| Spiramycin | Rhone-Poulene diarrhea | Cryptosporidial |
| Intraconazole-R51211 | Janssen-Pharm. | Histoplasmosis; cryptococcal meningitis |
| Trimetrexate | Warner-Lambert | PCP |
| Daunorubicin | NeXstar, Sequus | Kaposi's sarcoma |
| Recombinant Human Erythropoietin | Ortho Pharm. Corp. | Severe anemia assoc. with AZT therapy |
| Recombinant Human Growth Hormone | Serono | AIDS-related wasting, cachexia |
| Megestrol Acetate | Bristol-Myers Squibb | Treatment of anorexia assoc. W/AIDS |
| Testosterone | Alza, Smith Kline | AIDS-related wasting |
| Total Enteral Nutrition | Norwich Eaton Pharmaceuticals | Diarrhea and malabsorption related to AIDS |

When another agent having anti-HIV activity is included in the compressed tablet, it may be included within the same phase as the atazanavir sulfate or its formulation, i.e., as a monolithic tablet, or it may be included within another phase, i.e., a multi-layer tablet. When included in a monolithic tablet, the other agent may be blended intragranularly with the atazanavir sulfate or its formulation or added extragranularly. When included in a multi-layer tablet, the atazanavir sulfate is in one layer and the other agent (or agents) are in another layer, e.g., bilayer. Alternatively, when more than one other agent having anti-HIV activity is combined with atazanavir sulfate, e.g., ritonavir, emtricitabine and tenofovir, in a multilayer tablet, it may be desirable to separate certain agents by incorporating them in separate layers.

Also, although certain other agents having anti-HIV activity have been specifically disclosed, agents other than those specifically disclosed can be included in the compositions of the present invention. Also, more than one other agent having anti-HIV activity can be included in the compositions of the present invention.

## Claims

1. A compressed tablet comprising atazanavir sulfate, cobicistat, an acidifying agent and a precipitation retardant agent, wherein the precipitation retardant agent is selected from polyvinylpyrrolidone, polyvinylpyrrolidone-vinyl acetate, hydroxypropyl cellulose, hydoxypropyl methylcellulose, hydroxypropylmethylcellulose acetate succinate and mixtures thereof.

2. A compressed tablet according to claim 1 wherein the acidifying agent is selected from the group consisting of citric acid, tartaric acid, fumaric acid, ascorbic acid and mixtures thereof.

3. A compressed tablet according to claim 1 or claim 2 for use in a method of treating an HIV infection in a patient.

## Patentansprüche

1. Gepresste Tablette, umfassend Atazanavir-Sulfat, Cobicistat, ein Säuerungsmittel und ein Fällungsverzögerungsmittel, wobei das Fällungsverzögerungsmittel aus Polyvinylpyrrolidon, Polyvinylpyrrolidon-Vinylacetat, Hydroxypropylcellulose, Hydoxypropylmethylcellulose, Hydroxypropylmethylcellulose-Acetat-Succinat und Gemischen davon ausgewählt ist.

2. Gepresste Tablette gemäß Anspruch 1, wobei das Säuerungsmittel aus Citronensäure, Weinsäure, Fumarsäure, Ascorbinsäure und Gemischen davon ausgewählt ist.

3. Gepresste Tablette gemäß Anspruch 1 oder Anspruch 2 zur Verwendung in einem Verfahren zum Behandeln einer HIV-Infektion bei einem Patienten.

## Revendications

1. Comprimé comprenant du sulfate d'atazanavir, du cobicistat, un agent acidifiant et un agent retardant la précipitation, dans lequel l'agent retardant la précipitation est choisi parmi la polyvinylpyrrolidone, le polyvinylpyrrolidone-acétate de vinyle, l'hydroxypropylcellulose, l'hydroxypropylméthylcellulose, l'hydroxypropylméthylcellulose acétate succinate et leurs mélanges.

2. Comprimé selon la revendication 1, dans lequel l'agent acidifiant est choisi parmi l'acide citrique, l'acide tartrique, l'acide fumarique, l'acide ascorbique et leurs mélanges.

3. Comprimé selon la revendication 1 ou la revendication 2 pour une utilisation dans une méthode de traitement d'une infection par VIH chez un patient.
